# EUROPEAN PATENT APPLICATION

(11) **EP 1 389 477 A1**
(43) Date of publication of application: **18.02.2004**
(21) Application number: 03076338.7
(22) Date of filing: 06.05.2003
(51) Int. Cl.: A61M 25/00, A61B 18/02

(54) **Device for tip pressure monitoring for cryoablation catheters**

(30) Priority: 16.08.2002 US 222769
(71) Applicant: Cryocor, Inc., San Diego, California 92121 (US)
(72) Inventor: Ryba, Eric, San Diego, California 92122 (US)
(74) Representative: Shortt, Peter Bernard

(57) **Abstract**

A cryoablation catheter having a distal portion cooled by a refrigerant is described. The catheter includes a pressure sensor in the distal portion to monitor a pressure of the refrigerant. The monitored pressure may be used to determine the integrity of the catheter, both before and during a surgical procedure, so that the supply of refrigerant is interrupted if a leak is discovered. The monitored pressure may also be used in a feedback loop to control the performance of the cryoablation catheter.

## Description

### FIELD OF THE INVENTION

The present invention relates to devices and methods to monitor the pressure of a fluid within the tip of a catheter, and in particular of a cryoablation catheter.

### BACKGROUND OF THE INVENTION

Many surgical procedures require the removal of selected portions of diseased tissue within a patient's body cavity, as part of a treatment. The tissue may be removed by cryoablation, where the tip of a surgical catheter is brought in contact with the diseased tissue, and the tissue is cooled by the catheter to the point where the tissue becomes necrotic. For example, during certain cardiac procedures catheters or other devices may be inserted into a patient's vascular system and pushed through blood vessels to reach a desired location. Once the desired location has been reached, the tissue at that location may be treated using a device cooled in any of a variety of manners. For example, treatment of certain cardiac arrhythmias may include forming a conduction block of cryogenically ablated tissue between a source of improper contraction initiating signals and the left atrium. Surgical cryoablation may also be used on other medical fields, whenever removal of tissue must be accomplished with minimal blood loss and discomfort.

In many such procedures, the tip of the ablation catheter is cooled using a refrigeration process. For example, a Joule-Thompson refrigeration cycle may be employed, in which a refrigerant fluid at high pressure is expanded to a lower pressure, and also to a lower temperature. In many refrigeration processes the working fluid may be supplied to the catheter at a high pressure, through a system of tubes and hoses. The final temperature reached by the catheter and the cooling power of the device are related to the initial pressure of the refrigerant, as well as the composition of the refrigerant. In general, the cooling performance provided by the device may be controlled by changing the supply pressure of the refrigerant.

The refrigerant fluid used to cool the catheters may be selected to be non-toxic. However, even if the fluid is non toxic, leaks or spillage of the fluid within a body cavity of a patient can cause serious consequences. The refrigerant may be at a temperature that is damaging to the surrounding tissues, and a leak of the refrigerant at high pressure may cause mechanical tissue damage. Leaks within the body cavity should thus be stopped or prevented during the procedure.

In light of the above, an object of the present invention is to provide a catheter for cooling tissue in a body cavity, comprising a distal portion for insertion in the body cavity, a refrigerant supply line terminating in the distal portion to carry refrigerant from a supply to the distal portion, and a pressure sensor to monitor pressure of the refrigerant in the distal portion. Another object of the invention is to provide a method of operating a cryoablation catheter, comprising the steps of inserting a distal end of the catheter in a body cavity, supplying a refrigerant to the distal end via a supply line, and monitoring a pressure of the refrigerant with a pressure sensor disposed in the distal end.

### SUMMARY OF THE PREFERRED EMBODIMENTS

In accordance with the present invention, a cryoablation catheter for cooling a material includes a catheter body and a control unit. More specifically, the catheter body is tubular-shaped and is formed with a lumen. It also has an open proximal end. The distal end of the catheter body is, however, closed by a tip. Together, the tip and the catheter body form a chamber at the distal end of the catheter body.

A supply tube, which has a proximal end and a distal end, is formed with an orifice at its distal end. Operationally, the supply tube is positioned inside the lumen of the catheter body to establish a return line between the inner surface of the lumen in the catheter body and the outer surface of the supply tube. The orifice at the distal end of the supply tube is positioned inside the chamber adjacent the tip of the catheter body.

A fluid supply unit, such as bottles or canisters, is provided for introducing a fluid refrigerant into the supply tube. Specifically, this is accomplished through the proximal end of the supply tube. When introduced into the supply tube, the fluid refrigerant is at a first pressure. The fluid refrigerant then traverses through the lumen of the supply tube and exits through the orifice at the distal end of the tube. As the fluid refrigerant exits through the orifice, it expands as it enters into the chamber. More specifically, as intended for the present invention, the fluid refrigerant transitions from a liquid state to a gaseous state as it passes through the orifice. Through physics well known in the pertinent art, this transition generates a refrigeration effect that cools the tip of the catheter. Subsequently, the fluid refrigerant, now in a gaseous state, passes through the return line and exits the catheter at the proximal end of the catheter body.

An important aspect of the present invention is that a pressure sensor is mounted inside the chamber to measure a second pressure for the fluid refrigerant. Preferably, the pressure sensor is mounted in the chamber on the outside of the supply tube. As will be appreciated by the skilled artisan, however, the supply tube is formed with a lumen and the pressure sensor can alternatively be mounted inside the lumen of the supply tube. In either case, due to expansion of the fluid refrigerant, the second pressure of the fluid refrigerant as it is passing through the chamber, will be less than the initial, first pressure of the fluid refrigerant. As implied above, this pressure change will cool the tip of the catheter and, hence, the material. For this purpose, the tip is preferably made of a highly thermal conductive metal such as gold or platinum.

As indicated above, the catheter of the present invention includes a control unit. Preferably, this control unit is electronically connected to the pressure sensor for the purpose of monitoring the second pressure inside the chamber of the catheter. Additionally, the control unit will include a comparator for comparing this second pressure with a predetermined reference pressure. As a result of this comparison, the control unit is able to create an error signal which can be used to vary the first pressure. In accordance with standard closed loop feedback control techniques, the control unit will vary the first pressure on the fluid refrigerant to make the error signal a nullity. As an added feature, the catheter of the present invention can include an alarm. Specifically, the alarm is responsive to the error signal and will activate a shut down mechanism to prevent passage of the fluid refrigerant through the catheter whenever the error signal attains a predetermined value.

In an alternate embodiment of the present invention, the catheter of the present invention can include a temperature sensor. If included, the temperature sensor is mounted inside the chamber to measure a temperature for the tip. In this case the control unit is connected to the temperature sensor to monitor the temperature of the tip. The comparator in the control unit then compares the temperature of the tip with a reference temperature to create an error signal. Again, using well known closed loop feedback control techniques, this error signal is used to vary the first pressure in a manner that will make the error signal a nullity.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a side elevation view showing an exemplary embodiment of the cryoablation catheter with pressure measuring apparatus according to the present invention;
Fig. 2 shows a cross-sectional view of the pressure measuring apparatus as seen along the line 2-2 in Fig. 1; and
Fig. 3 is a diagram of a closed loop feedback control system for use in controlling the catheter of the present invention.

### DESCRIPTION OF THE PREFERRED EMBODIMENT

The present invention may be further understood with reference to the following description and the appended drawings, wherein like elements are referred to with the same reference numerals.

Cryoablation catheters of the type addressed by the present invention are typically used to remove diseased tissue located in a body cavity of the patient. During the procedure, the distal end of the catheter is inserted in the patient's body cavity, for example a vein or artery of the vascular system. The catheter is then pushed towards the site of the diseased tissue. Once the diseased tissue is reached, the tip of the catheter is placed in contact with the tissue, and the temperature of the tip is lowered. The tissue in contact with the tip is also cooled, to the point where it freezes and becomes necrotic. The amount of tissue ablated by this procedure can be controlled by varying the temperature and cooling power of the catheter.

The exemplary catheter according to embodiments of the present invention uses a Joule-Thompson refrigeration cycle to cool the tip. In this cycle, a high pressure refrigerant is supplied to the tip region of the catheter, where it is expanded through an orifice or similar device, so that the refrigerant's pressure and temperature drop. The exemplary catheter includes a tip pressure sensing apparatus that monitors the pressure of the refrigerant being supplied to the distal end of the catheter. As will be described in greater detail below, the sensing apparatus may include a pressure sensor located in the refrigerant supply tube, near the tip of the catheter. The high pressure present in the catheter before the refrigerant is expanded is of particular interest, since it provides an indication of the performance and integrity of the catheter. The pressure sensor may thus be placed in the refrigerant supply tube, upstream of the orifice used to expand the refrigerant.

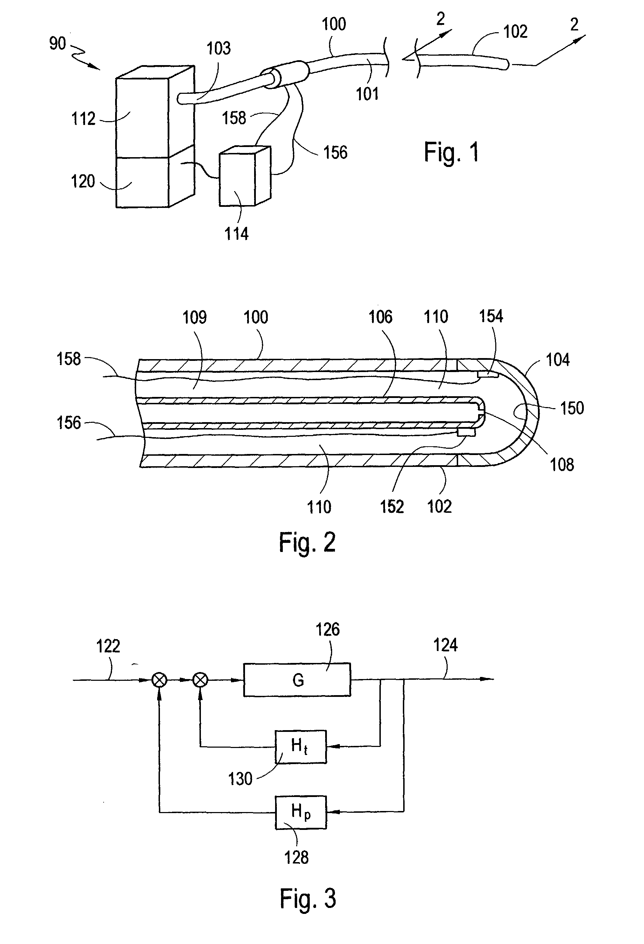

A cryoablation apparatus, generally designated 90, is shown in Fig. 1. In this exemplary embodiment according to the present invention, the cryoablation apparatus 90 is used to remove diseased tissue from a body cavity, for example from within a patient's vascular system. Although a Joule-Thompson refrigeration cycle may be used to cool the operative surfaces of the catheter, the preferred operation of the present invention involves a fluid phase change from liquid to gas, in addition to the expansion effects of a Joule-Thompson cycle. Cryoablation apparatus 90 includes a catheter 100 having a distal end 102 that is inserted into the body cavity, and a proximal end 103 that remains outside of the patient's body. Catheter 100 may be of conventional configuration, and may include the necessary elements to expand a refrigerant gas near the distal end 102.

As shown in Fig. 1 and Fig. 2, catheter 100 includes a catheter body 101 that terminates with a tip section 104 at the distal end 102. At the opposite end, the proximal end 103 of catheter 100 may be connected to a refrigerant supply unit 112. More specifically, a refrigerant supply tube 106 (see Fig. 2) may be connected to the refrigerant supply unit 112, and extends through a central lumen 109 of catheter 100 to near the tip 104. A return line 110 may also be located within catheter 100, to return the expanded refrigerant to the refrigerant supply unit 112. For example, return line 110 may be formed by the open spaces of lumen 109 within catheter 100. In different embodiments, the used refrigerant may be recycled or may be disposed of separately from the supply unit 112. Refrigerant supply unit 112 may include gas storage bottles, compressors, or any other elements necessary for providing refrigerant under pressure. A control unit 120 may also be included to control the pressure of the refrigerant provided by the refrigerant supply unit 112, and may include valves, pressure regulators, and the like.

Figure 2 shows a more detailed view of the distal end 102 of catheter 100. The exemplary embodiment shown in the drawing includes a refrigerant supply tube 106 with an orifice 108 formed near the tip 104. The high pressure refrigerant flows through supply tube 106, and is expanded through orifice 108, so that its pressure and temperature are lowered. The cold, expanded refrigerant impinges on the inner side of operative surface 150, which is thereby cooled. Since operative surface 150 is preferably made of a heat conductor such as gold or platinum, its outer surface is also cooled, and may be used to treat diseased tissue. A temperature sensor 154 may be placed on or near the operative surface 150 to monitor its temperature. Temperature sensor 154 may be connected to a sensing unit 114 through temperature sensor wires 158, or through a wireless connection.

Catheter 100 includes a pressure measuring system that monitors the pressure of the refrigerant in the distal portion 102. The pressure measuring system may include a pressure sensor 152 that is placed on the outside of supply tube 106 to measure the refrigerant pressure. Pressure sensor 152 may be connected to a pressure sensor wire 156 that extends the length of the catheter 100, to the proximal end 103, and connects to sensing unit 114. Other types of connections may be made between the pressure sensor 152 and the sensing unit 114, such as a wireless connection. Sensing unit 114 may include a display of the measured parameters, and may include a processor to evaluate the pressures and temperatures received from the respective sensors.

The presence of pressure sensor 152 within catheter 100 can be advantageously used for several purposes. For example, monitoring the refrigerant pressure in the catheter 100 can give an early indication of the existence of a leak somewhere within catheter 100. Catheter 100 forms a sealed system with supply unit 112, such that if the pressure of the refrigerant supplied by the supply unit 112 is known, it is possible to determine from the pressure measured by pressure sensor 152 whether the sealed system is leaking. This is an important benefit of the invention, because if a leak is detected during a procedure, the supply of refrigerant may be shut off, thus preventing damage to healthy tissues of the patient that could be caused by the leaking refrigerant. A leak may cause tissue damage even if the refrigerant fluid is non-toxic, due to the temperature of the refrigerant, or the pressure exerted on tissue by the leaking fluid.

In one exemplary embodiment, the pressure monitoring signal may be received by the sensing unit 114, which processes it to determine if a leak is occurring. If a leak is determined to be occurring, the control unit 120 may shut off supply of refrigerant from supply unit 112, so that no additional refrigerant is leaked. The existence of a leak may be determined, for example, by comparing the supply pressure of the refrigerant to the pressure monitored by sensor 152, and determining if the result falls within predetermined safety limits. The functions of sensing unit 114 and control unit 120 may be divided between several connected units, or may be combined into one single processing unit, without departing from the scope of the present invention. In the embodiment shown in Fig. 1, sensing unit 114 and control unit 120 are connected so that they can exchange data.

Pressure sensor 152 may also be used to perform an integrity check of the entire cryoablation catheter 100 prior to use in a surgical procedure. For example, before introducing the catheter 100 into the patient's body cavity, a "pump down" may be performed to test the catheter 100. In this procedure, a fluid at a known pressure may be introduced into the supply tube 106 of catheter 100. The pressure monitored by pressure sensor 152 near the tip 104 may be compared to the known pressure, and the results may be correlated to guidelines that indicate whether a leak is probable. The values of the guidelines may be determined experimentally or computationally for the catheter in question. If the integrity check indicates that no leaks are present, the procedure may continue normally. If the probability of a leak is indicated, the procedure may be suspended, without the patient ever being put at risk from the leaking refrigerant.

The performance of the cryoablation apparatus 90 may also be improved by using data from pressure sensor 152 in a feedback loop. The temperature of operating surface 150 and the cooling power of the catheter 100 can be correlated to the pressure of the unexpanded refrigerant at the tip 104, for a given refrigerant flow rate. Monitoring the tip pressure thus allows the user to monitor the cooling performance of catheter 100. For example, the pressure measured by sensor 152 may be compared to known pressures required to perform various procedures. If the pressure reported by pressure sensor 152 is too low or too high, sensing unit 114 and control unit 120 may cause supply unit 112 to provide refrigerant at a correspondingly higher or lower pressure, to obtain the desired performance.

The control for catheter 100 is perhaps best appreciated by reference to Fig. 3. In Fig. 3, a typical closed loop feedback control diagram is provided

For the present invention, the command input 122 can be taken to be the pressure of the refrigerant as it is being introduced into the supply tube 106 from the supply unit 112. The output 124 will then be a measure of a physical characteristic of the refrigerant as it passes through the tip section 104. For the present invention, this measure can be either a pressure, as measured by pressure sensor 152, or a temperature, as measured by temperature sensor 154. The change between the input 122 and the output 124 will be caused by the dynamic element 126 (G), of the apparatus 90.

As envisioned by the present invention, a pressure measurement taken by the pressure sensor 152 represents the feedback element 128 (Hₚ). Similarly, a temperature measurement taken by the temperature sensor 154 represents the feedback element 130 (H_{T}). As shown in Fig. 1, these measurements are transmitted respectively via the pressure sensor wire 156 and the temperature sensor wire 158 through the sensing unit 114 to the control unit 120. As indicated in Fig. 3, the feedback of pressure (Hₚ), and the feedback of temperature (H_{T}), can be compared separately, or jointly, with reference selectors in the control unit 120. This comparison is made to generate respective error signals. In a manner well known in the pertinent art, the error signals that are so generated can be used to vary the input 122 in a manner that will maintain the desired output 124.

A more direct measure of the performance of cryoablation apparatus 90 may be obtained by using the pressure sensor 152 in conjunction with one or more temperature sensors 154. The temperature of operative surface 150 is affected by the transfer of heat from bodily fluids and tissues, such as blood flowing past the tip 104. The temperature of tip 104 mapped using temperature sensors 154 and the pressure of the refrigerant measured by pressure sensor 152 may be used to determine the effectiveness of the treatment. For example, the temperature distribution in the tissues surrounding tip 104 may be determined, to ensure that the tissue is cooled uniformly. A controlled delivery of cryogenic therapy may be performed by monitoring the pressure of the refrigerant and the temperature of the tip 104, so that excessive or insufficient cooling does not take place. To take advantage of the benefits afforded by this type of feedback, refrigerant supply unit 112 may include valves or other devices that allow it to supply refrigerant at different pressures, in response to commands from the control unit 120 and sensing unit 114.

According to exemplary embodiments of the present invention, the pressure sensor 152 may be formed by including a separate tube within the central lumen of catheter 100, in proximity of tip 104. As will be appreciated by the skilled artisan, any type pressure sensor well known in the pertinent art can be used for this purpose.

During operation of the cryoablation apparatus 90, monitoring of the tip pressure may be carried out to achieve multiple objectives. Before the start of the surgical procedure, the catheter 100 and associated tubing may be tested for leaks by applying a known pressure to the refrigerant, and measuring the pressure at the tip 104 with pressure sensor 152. As indicated above, the procedure will not begin if a leak is discovered. During the surgical procedure, the tip pressure may be monitored continuously to determine if a leak begins to form. If a drop in the monitored pressure indicates that a leak may exist, the procedure may be discontinued before the patient can be harmed by the leaking refrigerant. The performance of the cryoablation apparatus 90 may be monitored and adjusted during the surgical procedure, by monitoring the tip pressure in a feedback loop. As described above, monitoring the pressure and in some cases the temperature at the tip of the catheter 100 allows the operator to apply the appropriate amount of cooling to the diseased tissue. These functions may be performed concurrently or separately, depending on the sophistication of the control and sensing units.

In the preceding specification, the present invention has been described with reference to specific exemplary embodiments thereof. It will, however, be evident that various modifications and changes may be made thereto without departing from the broadest spirit and scope of the present invention as set forth in the claims that follow. The specification and drawings are accordingly to be regarded in an illustrative rather than restrictive sense. For example, while the invention has been described for use in a Joule Thompson cryoablation catheter, the invention may be used in different types of catheters in which a high pressure fluid is introduced.

While the particular Tip Pressure Monitoring for Cryoablation Catheters as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A cryoablation catheter for cooling a material, which comprises:
a catheter body formed with a lumen, said catheter body having an open proximal end and having a closed distal end, said closed distal end defining a tip for said catheter and forming a chamber in said catheter body;
a supply tube having a proximal end and a distal end with an orifice formed at said distal end, said supply tube being positioned in said lumen of said catheter to establish a return line therebetween, with said orifice positioned in said chamber adjacent said tip of said catheter body;
a means for introducing a fluid refrigerant into said supply tube through said proximal end thereof at a first pressure, for expansion of the fluid refrigerant into said chamber through said orifice, for subsequent passage through said return line and out said proximal end of said catheter body; and
a pressure sensor mounted in said chamber for measuring a second pressure for the fluid refrigerant in said chamber as the fluid refrigerant passes therethrough to cool said tip and the material.

2. A catheter as recited in claim 1 wherein said supply tube is formed with a lumen and said pressure sensor is mounted in said lumen of said supply tube.

3. A catheter as recited in claim 1 wherein said tip is made of a metal selected from a group consisting of gold and platinum.

4. A catheter as recited in claim 1 further comprising a control unit which comprises:
a means connected to said pressure sensor for monitoring the second pressure;
a means for comparing the second pressure with a reference pressure to create an error signal; and
a means for varying the first pressure to make the error signal a nullity.

5. A catheter as recited in claim 4 further comprising an alarm means responsive to the error signal for shutting down said introducing means to prevent passage of the fluid refrigerant through said catheter when the error signal attains a predetermined value.

6. A catheter as recited in claim 1 further comprising a temperature sensor mounted in said chamber to measure a temperature for said tip.

7. A catheter as recited in claim 6 further comprising a control unit which comprises:
a means connected to said temperature sensor for monitoring the temperature of said tip;
ameans for comparing the temperature of said tip with a reference temperature to create an error signal; and
a means for varying the first pressure to make the error signal a nullity.

8. A catheter as recited in claim 1 further comprising a means for positioning said tip against the material to be cooled.

9. A catheter as recited in claim 1 wherein the material is living tissue.

10. A method for controlling a cryoablation catheter to cool a material, which comprises the steps of:
providing a catheter having a catheter body formed with a lumen, the catheter body having an open proximal end and having a closed distal end with the closed distal end defining a tip for the catheter and forming a chamber in the catheter body, the catheter further having a supply tube with a proximal end and a distal end with an orifice formed at the distal end, the supply tube being positioned in the lumen of the catheter to establish a return line therebetween, with the orifice positioned in the chamber adjacent the tip of the catheter body; and with a pressure sensor mounted in said chamber;
introducing a fluid refrigerant into said supply tube through said proximal end thereof at a first pressure, for expansion of the fluid refrigerant into said chamber through said orifice, for subsequent passage through said return line and out said proximal end of said catheter body;
using the pressure sensor for measuring a second pressure for the fluid refrigerant in said chamber as the fluid refrigerant passes therethrough to cool said tip and the material; and
maintaining the second pressure at a predetermined level to control the cooling of the material.

11. A method as recited in claim 10 wherein the supply tube is formed with a lumen and the pressure sensor is mounted in the lumen of the supply tube.

12. A method as recited in claim 10 wherein the tip is made of a metal selected from a group consisting of gold and platinum.

13. A method as recited in claim 10 wherein said maintaining step comprises the steps of:
monitoring the second pressure;
comparing the second pressure with a reference pressure to create an error signal; and
varying the first pressure to make the error signal a nullity.

14. A method as recited in claim 13 further comprising the step of activating an alarm means responsive to the error signal for shutting down said introducing step to prevent passage of the fluid refrigerant through the catheter when the error signal attains a predetermined value.

15. A method as recited in claim 13 further comprising the steps of:
mounting a temperature sensor in the chamber to measure a temperature for the tip;
monitoring the temperature of the tip;
comparing the temperature of the tip with a reference temperature to create an error signal; and
varying the first pressure to make the error signal a nullity.

16. A method as recited in claim 10 further comprising the step of positioning the tip against the material to be cooled, wherein the material is living tissue.
